# EUROPEAN PATENT APPLICATION

(11) **EP 4 585 569 A1**
(43) Date of publication of application: **16.07.2025**
(21) Application number: 25150696.0
(22) Date of filing: 08.01.2025
(51) Int. Cl.: C02F 1/36, A61L 2/025, C02F 103/00

(54) **WASTE MANAGEMENT SYSTEM WITH BUILT-IN PIEZOELECTRIC BASED INTERFACES FOR WATER RECOVERY AND STERILIZATION VIBRATION**

(30) Priority: 09.01.2024 US 202418408144
(71) Applicant: Hamilton Sundstrand Corporation, Charlotte, NC 28217-4578 (US)
(72) Inventor: MESA, Jorge Ramon, League City, TX (US)
(74) Representative: Dehns

(57) **Abstract**

A system is provided. The system includes: a receptacle (302); one or more piezoelectric transducers (315) included in the receptacle (302); and amplifier circuitry coupled to the one or more piezoelectric transducers (315) and configured to provide an alternating-current (AC) voltage signal to the one or more piezoelectric transducers (315), based on a target configuration associated with recovering water from a substance included in the receptacle (302). The one or more piezoelectric transducers (315) are configured to vibrate based on a magnitude and a frequency of the AC voltage signal. The system is configured to extract a mist form of the water from the substance, based on the vibration of the one or more piezoelectric transducers (315).

## Description

### BACKGROUND

The present disclosure relates to water recovery, and more, particularly to a water recovery system utilizing a piezoelectric based interfaces.

Human waste such as, for example, feces, menses, and vomit may contain up to 75% water. In some zero-gravity toilets (e.g., space toilets) usable in a weightless environment, human waste is stored in fecal bags which may reside in a canister of the space toilet. Human generated trash, for example, may contain up to 30% water. In some cases, once a canister aboard a space station or spacecraft is filled with human waste and/or human generated trash and has been sealed, the canister is temporarily stored and eventually disposed of upon reentry into Earth's atmosphere.

### BRIEF DESCRIPTION

According to an aspect of the disclosure, a system is provided. The system includes: a receptacle; one or more piezoelectric transducers included in the receptacle; and amplifier circuitry coupled to the one or more piezoelectric transducers and configured to provide an alternating-current (AC) voltage signal to the one or more piezoelectric transducers, based on a target configuration associated with recovering water from a substance included in the receptacle. The one or more piezoelectric transducers are configured to vibrate based on a magnitude and a frequency of the AC voltage signal. The system is configured to extract a mist form of the water from the substance, based on the vibration of the one or more piezoelectric transducers.

In one or more embodiments: one or more flexible bladders configured to store the substance and including a plurality of pores, wherein the one or more flexible bladders are configured to retain a liquid form of water, based on a pore size of the plurality of pores, wherein the system is configured to extract the mist form of the water via the plurality of pores, based on the vibration of the one or more piezoelectric transducers.

In any one or combination of the embodiments disclosed herein, the one or more piezoelectric transducers are removably coupled to one or more inner surfaces of the receptacle and are in contact with one or more surfaces of the one or more flexible bladders.

In any one or combination of the embodiments disclosed herein, the one or more piezoelectric transducers are integrated with a material of the one or more flexible bladders.

In any one or combination of the embodiments disclosed herein: the vibration of the one or more piezoelectric transducers separates the water from the substance; and at least a portion of the water separated from the substance based on the vibration of the one or more piezoelectric transducers is in a mist form including water droplets.

In any one or combination of the embodiments disclosed herein, the system may further include one or more piezoelectric assemblies included in the receptacle, wherein: the one or more piezoelectric assemblies include the one or more piezoelectric transducers; the one or more piezoelectric assemblies include a flexible material; and the one or more flexible bladders are between the one or more piezoelectric assemblies and at least one inner surface of the receptacle.

In any one or combination of the embodiments disclosed herein, the system may further include one or more piezoelectric assemblies included in the receptacle, wherein: the one or more piezoelectric assemblies include the one or more piezoelectric transducers; the one or more piezoelectric assemblies include a flexible material; and at least one first flexible bladder of the one or more flexible bladders is between the one or more piezoelectric assemblies and at least one inner surface of the receptacle.

In any one or combination of the embodiments disclosed herein: at least one second flexible bladder of the one or more flexible bladders is between the one or more piezoelectric assemblies and at least one second inner surface of the receptacle; or the at least one second flexible bladder is between the one or more piezoelectric assemblies and one or more second piezoelectric assemblies included in the receptacle, wherein the one or more second piezoelectric assemblies include one or more second piezoelectric transducers.

In any one or combination of the embodiments disclosed herein, the substance includes at least one of urine; urine brine; feces; trash; and wastewater.

In any one or combination of the embodiments disclosed herein, the system further includes: at least one storage container; one or more ports coupled between the receptacle and the at least one storage container; and one or more second piezoelectric transducers coupled to the one or more ports. In some aspects, the system is configured to transfer the recovered water to at least one storage container via the one or more ports; the amplifier circuitry is configured to provide a second AC voltage signal to the one or more second piezoelectric transducers, based on the target configuration, where the one or more second piezoelectric transducers are configured to vibrate based on a second magnitude and a second frequency of the AC voltage signal; and the vibration of the one or more second piezoelectric transducers sterilizes at least a portion of the recovered water.

In any one or combination of the embodiments disclosed herein, the target configuration is based on one or more characteristics of the substance, the one or more characteristics including at least one of: a type of the substance; and an estimated water content associated with the substance.

In any one or combination of the embodiments disclosed herein: the target configuration is based on a target sterilization level associated with sterilizing at least a portion of the recovered water; and the vibration of the one or more piezoelectric transducers sterilizes at least the portion of the water.

In any one or combination of the embodiments disclosed herein: the target configuration is based on a target sterilization level associated with sterilizing at least a portion of solid matter included in the substance; and the vibration of the one or more piezoelectric transducers sterilizes at least the portion of the solid matter.

In any one or combination of the embodiments disclosed herein, the system is configured for recovering the water under partial gravity, microgravity, or zero gravity conditions.

According to an aspect of the disclosure, a receptacle is provided including: one or more piezoelectric transducers; and amplifier circuitry coupled to the one or more piezoelectric transducers and configured to provide an alternating-current (AC) voltage signal to the one or more piezoelectric transducers, based on a target configuration associated with recovering water from a substance included in the receptacle. The one or more piezoelectric transducers are configured to vibrate based on a magnitude and a frequency of the AC voltage signal. The receptacle is configured to extract a mist form of the water from the substance, based on the vibration of the one or more piezoelectric transducers.

In any one or combination of the embodiments disclosed herein, the receptacle further includes: one or more flexible bladders configured to store the substance and including a plurality of pores, wherein the one or more flexible bladders are configured to retain a liquid form of water, based on a pore size of the plurality of pores, wherein the receptacle is configured to extract the mist form of the water via the plurality of pores, based on the vibration of the one or more piezoelectric transducers.

In any one or combination of the embodiments disclosed herein, the one or more piezoelectric transducers are removably coupled to one or more inner surfaces of the receptacle and are in contact with one or more surfaces of the one or more flexible bladders.

In any one or combination of the embodiments disclosed herein, the one or more piezoelectric transducers are integrated with a material of the one or more flexible bladders.

In any one or combination of the embodiments disclosed herein: the vibration of the one or more piezoelectric transducers separates the water from the substance; and at least a portion of the water separated from the substance based on the vibration of the one or more piezoelectric transducers is in a mist form including water droplets.

According to an aspect of the disclosure a method includes: determining a target configuration associated with recovering water from a substance included in a receptacle; determining, based on the target configuration, a magnitude and a frequency of an alternating-current (AC) voltage signal to be applied to one or more piezoelectric transducers of a plurality of piezoelectric transducers, wherein the plurality of piezoelectric transducers are included in the receptacle; providing, by amplifier circuitry, the AC voltage signal to the one or more piezoelectric transducers, based on the target configuration, wherein the one or more piezoelectric transducers vibrate based on the magnitude and the frequency of the AC voltage signal, wherein the vibration of the one or more piezoelectric transducers separates the water from the substance; and extracting, to at least one storage container, at least a portion of the water separated from the substance.

Additional features and advantages are realized through the techniques of the present disclosure. Other embodiments and aspects of the disclosure are described in detail herein and are considered a part of the claimed technical concept. For a better understanding of the disclosure with the advantages and the features, refer to the description and to the drawings.

### BRIEF DESCRIPTION OF THE DRAWINGS

For a more complete understanding of this disclosure, reference is now made to the following brief description, taken in connection with the accompanying drawings and detailed description, wherein like reference numerals represent like parts.
FIG. 1 illustrates an example of a piezoelectric system in accordance with one or more embodiments of the present disclosure.
FIG. 2 illustrates an example of a piezoelectric assembly in accordance with one or more embodiments of the present disclosure.
FIG. 3 illustrates an example of a system supportive of water recovery and sterilization in accordance with one or more embodiments of the present disclosure.
FIG. 4 illustrates an example of a system supportive of water recovery and sterilization in accordance with one or more embodiments of the present disclosure.
FIG. 5 illustrates an example flowchart of a method in accordance with one or more embodiments of the present disclosure.
FIGS. 6A through 6C illustrate examples of a water recovery system supportive of water recovery and sterilization in accordance with one or more embodiments of the present disclosure.
FIG. 7 illustrates an example of a liquid processor assembly supportive of water recovery and sterilization in accordance with one or more embodiments of the present disclosure.

### DETAILED DESCRIPTION

A detailed description of one or more embodiments of the disclosed apparatus and method are presented herein by way of exemplification and not limitation with reference to the Figures.

It is an immediate NASA goal to recover 90% of water from human waste deposited in a zero-gravity toilet (e.g., space toilet). However, the international space station (ISS) and other planned space stations are absent a fecal processor capable of recovering such water.

Furthermore, with proposed plans for planetary habitats and exploration of the Moon, Mars, and the like, equipping exploration vehicles (e.g., lunar terrain vehicles) with relatively simple and compacted toilet capabilities for disposing human waste on the planetary surface are desired, in which the human waste is organically and chemical inert. Such target conditions may pose a challenge, for example, since in some space exploration applications, human waste is stored and disposed of untouched (e.g., unprocessed). Accordingly, for example, if left unchecked and untouched, under some conditions, microbes in the human waste may multiply relatively fast.

Techniques are desired which may prevent the disposal of human waste (e.g., as stored in sealed bags or canisters) on the lunar surface, and further, support the recovery of water from human waste and sterilization of the recovered water. Some other techniques for waste processing may involve and/or combine techniques such as, for example, evaporation, vacuuming, chemical treatment, separation, and UV light. Some other techniques for water recovery and sterilization may involve heat, which may be relatively time consuming, power intensive, and concept of operations (CONOPS) sensitive.

Human waste is currently stored in fecal bags inside of a 'space toilet' canister. In disposing of the fecal bags, opportunities for recapturing the high-water content of human waste are lost. Long term human space exploration may be improved through techniques described herein for recovering water from human waste, sterilization of the water, and sterilization of remaining solids.

The systems and techniques for water recovery and sterilization as described herein may support reduced over costs associated with space stations, planetary habitats, space exploration vehicles, and the like. For example, the systems and techniques described herein (e.g., through the recovery of water) may extend the lifespan of a space exploration mission or planetary mission by increasing the available water supply.

According to one or more embodiments of the present disclosure, a piezoelectric assembly is described which may include a lightweight, low power, and relatively small (e.g., small volume) assembly that includes a plurality of piezoelectric transducers. Example aspects of the piezoelectric assembly and a water recovery system including the piezoelectric assembly are described with reference to the following figures.

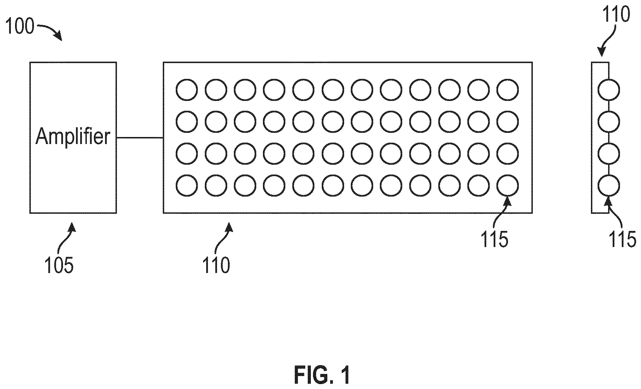

FIG. 1 illustrates an example of a piezoelectric system 100 in accordance with one or more embodiments of the present disclosure.

According to one or more embodiments of the present disclosure, the piezoelectric system 100 may include an amplifier 105 and a piezoelectric assembly 110. In some embodiments, the amplifier 105 is a signal generator amplifier capable of generating a signal (e.g., an alternating-current (AC) voltage signal) and providing the signal to piezoelectric transducers 115 included in the piezoelectric assembly 110. In some examples, the signal may be a high current, high voltage (i.e., high voltage magnitude), and high frequency signal, for example, a high frequency sine wave. According to one or more embodiments of the present disclosure, the piezoelectric assembly 110 and piezoelectric transducers 115 may include piezo-based assemblies rated for surviving the effects of thermal, radiation, vacuum, micro-g, and other conditions associated with space exploration,

In some aspects, the amplifier 105 may provide the signal to the piezoelectric assembly 110 (and included piezoelectric transducers 115) and/or other piezoelectric assemblies 110 (and included piezoelectric transducers 115) (not illustrated) included in the piezoelectric system 100. In some embodiments, the piezoelectric system 100 may include multiple amplifiers 105, and each amplifier 105 is capable of generating and providing a signal according to target signal parameters (e.g., current level, voltage level, frequency, signal type, and the like) to one or more piezoelectric assemblies 110.

In some examples, the frequency of the signal provided by the amplifier 105 may be in an ultrasonic frequency range. For example, the frequency of the signal may be in a range (e.g., 1 Mhz to 3 Mhz) supportive of water extraction applications. In some examples, the frequency of the signal may be in a range (e.g., about 10 kHZ to about 500 kHz) supportive of sterilization applications of solids such as, for example, dust, regolith, or dirt, or in some cases, solids remaining from the water extraction of wastewater (e.g., from urine, brine, feces, vomit, menses, trash, or the like). However, aspects of the present disclosure are not limited thereto, and the amplifier 105 may be capable of providing signals of any current, voltage, and/or frequency suitable for driving the piezoelectric transducers 115 in accordance with the water recovery techniques supported by the present disclosure. For example, aspects of the present disclosure support configuring the current, voltage, and/or frequency based on particle size, material content, density of a solid to be extracted from a substance (e.g., wastewater, feces, trash, and the like) and/or sterilized. In some embodiments, the amplifier 105 may be a standalone device. In some other embodiments, the amplifier 105 may be integrated in a signal generator, a computing device (e.g., a computing device 308 later described herein), or other electronic circuitry associated with the piezoelectric system 100.

Example aspects of the piezoelectric assembly 110 are described herein. The piezoelectric assembly 110 may be formed of a material such that the piezoelectric assembly 110 is relatively lightweight. In some aspects as will be described herein, the piezoelectric assembly 110 may be lightweight, consume a relatively low amount of power, and relatively small (e.g., small volume) such that the piezoelectric assembly 110 may be integrated with a zero-gravity toilet (e.g., space toilet, low gravity toilet), a receptacle for storing human waste or human generated trash, a space suit, or other structure.

The piezoelectric assembly 110 may include piezoelectric transducers 115 (also referred to herein as piezoelectric sensors or piezos). In some embodiments, the piezoelectric transducers 115 are disposed in an array configuration. However, aspects of the present disclosure are not limited thereto, and the piezoelectric transducers 115 may be disposed or arranged according to any suitable pattern supportive of the techniques described herein.

In some examples, the piezoelectric transducers 115 are disposed in the piezoelectric assembly 110 and arranged such that the piezoelectric transducers 115 are spaced apart from each other by a distance in any suitable direction (e.g., X direction and/or Y direction with respect to an XY plane of the piezoelectric assembly 110). In some examples, the diameter of each piezoelectric transducer 115 may be about 0.25 inches. In some embodiments, the center to center (C-C) distance from a piezoelectric transducer 115 to another piezoelectric transducer 115 may be about 0.375 inches, and the edge to edge (e-e) distance from a piezoelectric transducer 115 to another piezoelectric transducer 115 may be about 0.125 inches. However, aspects of the present disclosure are not limited thereto, and the systems and techniques described herein support maintaining or adjusting the diameters of the piezoelectric transducers 115 and/or distances between piezoelectric transducers 115 based on a target application (e.g., water recovery and extraction, solids sterilization, water sterilization, drying, or the like) of the piezoelectric assembly 110, available surface area of the piezoelectric assembly 110, and the like.

The piezoelectric transducers 115 are configured to contract or expand based on a voltage signal applied (e.g., by the amplifier 105) to the piezoelectric transducers 115. In some aspects, through the contraction and expansion of the piezoelectric transducers 115, the piezoelectric assembly 110 may support features for recovering a fluid from a substance (e.g., urine, urine brine, feces, trash, a solid matter, a partially solid matter, and the like) included in a receptacle of a zero-gravity toilet or a receptacle in which bags including the substance are stored. For example, the piezoelectric assembly 110 may support recovery of water from the substance.

In some embodiments, by using an amplifier 105, the piezoelectric transducers 115 may vibrate at a relatively high frequency supportive of removing water from the substance. The piezoelectric transducers 115 may be interwoven in the fabric, bladder, bag, or membrane containing a substance (e.g., urine, feces, waste, or the like) to be processed. In some embodiments, the piezoelectric transducers 115 (with or without a corresponding piezoelectric assembly 110) may be electrically connected or plugged into the amplifier 105. In some cases, the amplifier 105 may be integrated with an inner surface of a receptacle (e.g., a canister) or a toilet assembly.

In some embodiments, the piezoelectric system 100 may include a piezoelectric assembly 110 integrated with an external surface of a receptacle including substance-filled bags (e.g., filed with urine, feces, waste, or the like), and the piezoelectric system 100 may support the extraction of water from and drying of the bags through the vibration of the piezoelectric transducers 115.

In accordance with one or more embodiments of the present disclosure, the piezoelectric system 100 and piezoelectric assemblies 110 may provide a solution for water recovery and sterilization that is a resilient and long lasting, has a relatively low complexity for development, maintenance, and operation, and has relatively low overhead cost, as components of the piezoelectric system 100 have high technology readiness level (TRL). For example, the piezoelectric transducers 115 and piezoelectric assemblies 110 implemented in association with the piezoelectric system 100 described herein may be rated for performance under conditions (e.g., thermal, radiation, vacuum, microgravity, zero gravity, and the like) associated with aircrafts, space satellites, space exploration, planetary exploration, and the like. In an example, the piezoelectric transducers 115 and piezoelectric assemblies 110 implemented may have a TRL 9 rating (e.g., system proven and ready for full commercial deployment).

According to one or more embodiments of the present disclosure, the piezoelectric system 100 may control the vibration of the piezoelectric transducers 115 such that the vibration is capable of reducing the microbial count of a contaminated area, in some examples, by more than 5000 times.

It is to be understood that the techniques described herein are not limited to recovery of fluid from human waste. For example, the techniques described herein may support the recovery of water from trash, wastewater (e.g., laundry wastewater), or the like. For example, the piezoelectric assembly 110 and included piezoelectric transducers 115 support a piezo interface configured to provide a forced vibration that may remove water from a substance (e.g., urine, urine brine, feces, trash, wastewater, and the like) as supported by aspects of the present disclosure.

In some embodiments, the techniques described herein include using a piezoelectric interface to force vibration that extracts the water content from waste inside of a microporous bag, in the form of a relative cold mist. The techniques described herein may include (e.g., subsequent to extraction of the water or at the same time) disinfecting the water and/or solid contents. The techniques described herein may be implemented standalone and/or in combination other water extraction and sterilization techniques (e.g., evaporation, vacuum, flushing, filtration, or the like).

The piezoelectric transducers 115 are capable of converting one form of energy into another. For example, with respect to piezoelectricity, the piezoelectric transducers 115 are capable of converting mechanical pressure or stress into electrical signals and reciprocally translating electrical signals into mechanical displacement.

In some embodiments, the piezoelectric transducers 115 may be of any suitable size (examples of which are described herein) supportive of the techniques described herein. For example, the piezoelectric transducers 115 may be of a size supportive of implementation in a zero-gravity toilet or in a receptacle for storing a substance (e.g., urine, urine brine, feces, trash, wastewater, and the like). The piezoelectric transducers 115 may be of a size supportive of effective vibration for water recovery and/or sterilization of the recovered water. However, aspects of the present disclosure are not limited thereto, and the piezoelectric transducers 115 may be of any size supportive of the techniques described herein.

In some embodiments, the piezoelectric assembly 110 may be a membrane. In some aspects, the membrane may be relatively thin to support effective integration with a structure (e.g., TCPS 301, TCPS 401 later described herein). In some embodiments, the piezoelectric assembly 110 may be a mesh structure. In some embodiments, the piezoelectric assembly 110 may be a plate assembly. In some aspects, the plate assembly may be perforated or indented so as to accommodate the piezoelectric transducers 115. In some embodiments, the piezoelectric assembly 110 may be formed of a fabric material and/or a non-fabric flexible material.

According to one or more embodiments of the present disclosure, the piezoelectric transducers 115 (and/or piezoelectric assembly 110) may be interwoven or integrated into a thin membrane, bladder, or bag associated with a receptacle (e.g., a receptacle 302 later described herein). In some embodiments, the piezoelectric assembly 110 may be implemented as an external add-in and be permanently coupled or detachably coupled to one or more inner surfaces of the receptacle. In some embodiments in which the piezoelectric assembly 110 is a membrane based microporous bag or bladder, a thickness range of the piezoelectric assembly 110 may be about 10 microns to about 100 microns, and in some examples, about 20 microns to about 60 microns (e.g., for wastewater processing). In some embodiments in which the piezoelectric assembly 110 is a plate assembly, a thickness range of the piezoelectric assembly 110 may be about 1/16 of an inch to about 1/8 of an inch. In some embodiments in which the piezoelectric assembly 110 is a mesh structure, a thickness range of the piezoelectric assembly 110 may be about 1/32 of an inch to about 1/64 of an inch.

Aspects of the present disclosure support modular implementations of the piezoelectric assembly 110. For example, in the case of a piezoelectric assembly 110 detachably couped to one or more inner surfaces of the receptacle, aspects of the present disclosure support modular replacement of a piezoelectric assembly 110 (e.g., among multiple piezoelectric assemblies 110) for cases in which piezoelectric transducers 115 of the piezoelectric assembly 110 break, malfunction, are relatively near a specified life expectancy, or the like.

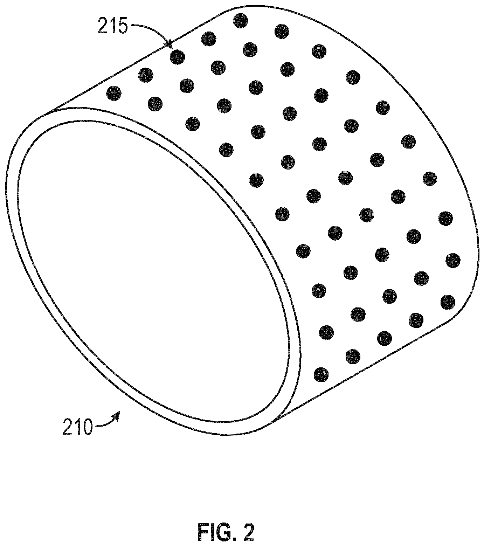

FIG. 2 illustrates an example of a piezoelectric assembly 210 in accordance with one or more embodiments of the present disclosure. The piezoelectric assembly 210 includes aspects of piezoelectric assembly 110 described herein, and repeated descriptions of like elements are omitted for brevity

In the example of FIG. 2, the piezoelectric assembly 210 is of a cylindrical configuration. In some embodiments, the piezoelectric assembly 210 may support applications for cylindrical (and partially cylindrical) shaped end-products such as, for example, a liner of a receptacle.

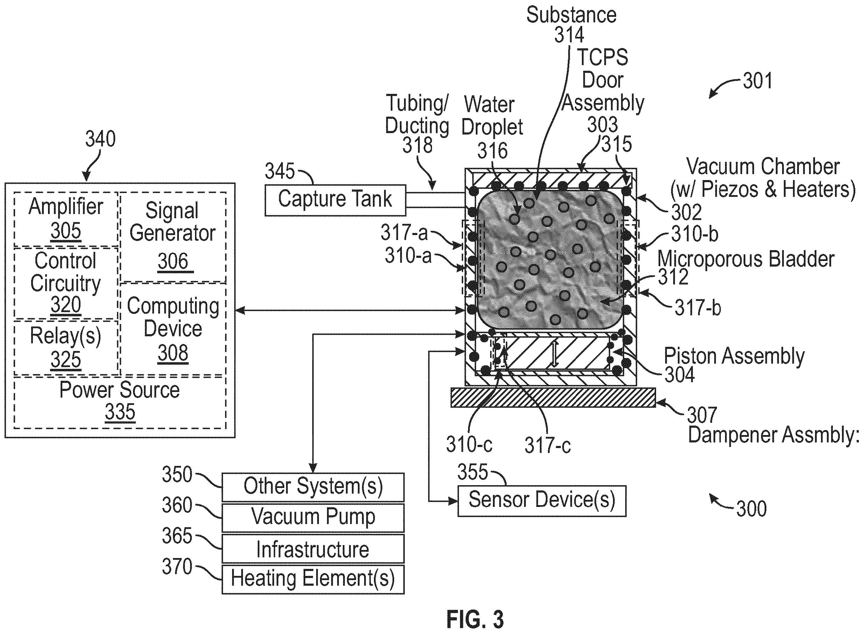

FIG. 3 illustrates an example of a water recovery system 300 supportive of water recovery and sterilization in accordance with one or more embodiments of the present disclosure. The water recovery system 300 includes aspects of piezoelectric system 100 described herein, and repeated descriptions of like elements are omitted for brevity. The water recovery system 300 may include a trash compaction and processing system (TCPS) 301.

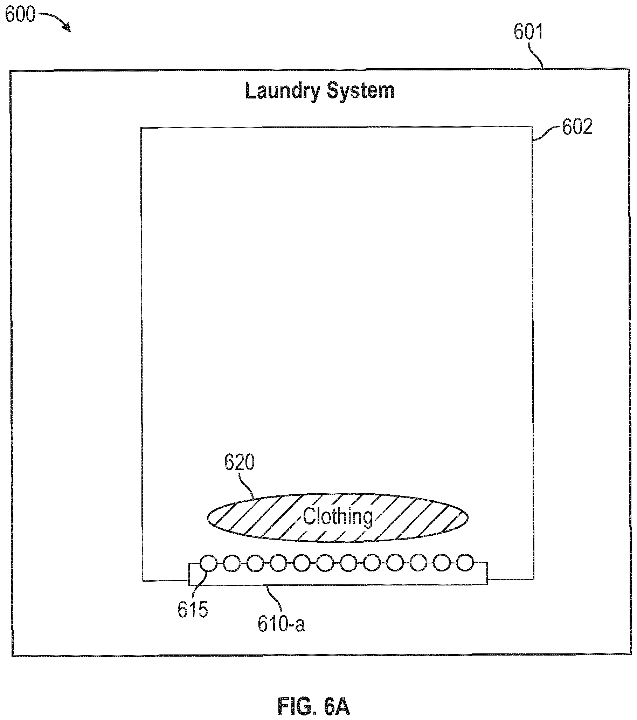

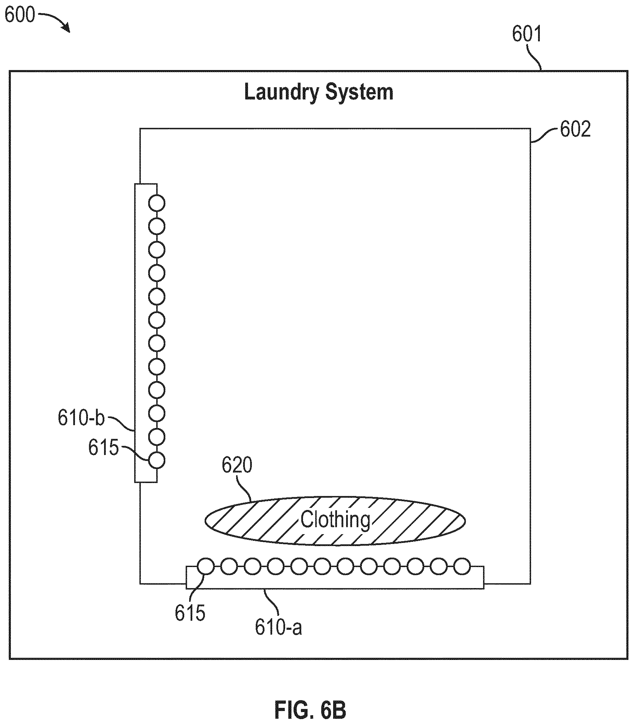

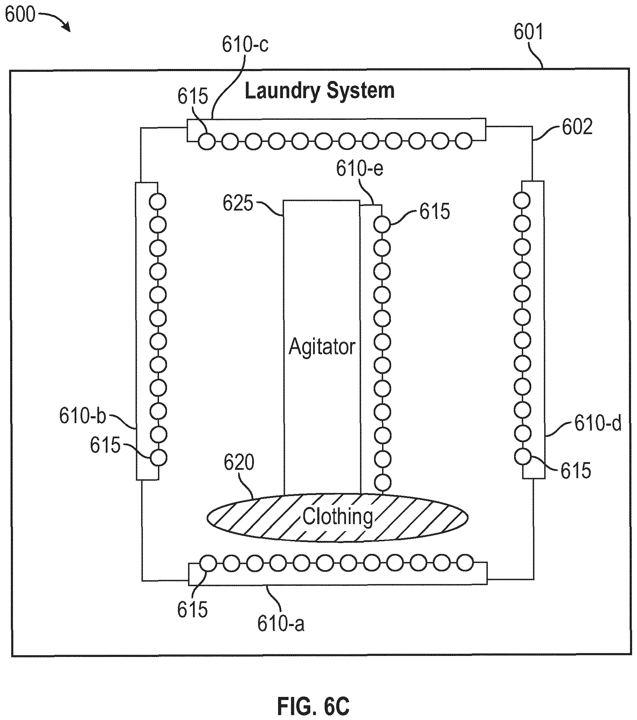

However, aspects of the present disclosure are not limited thereto, and the water recovery and sterilization techniques described in association with the water recovery system 300 may be implemented for water recovery and sterilization from wastewater associated with other systems (e.g., laundry systems for use in a zero-gravity or near zero-gravity environment). For example, the water recovery system 300 may be implemented in association with a laundry system later described with reference to FIG. 6.

The water recovery system 300 may support features for waste management as described herein and may also be referred to as a waste management system. The features supported by the water recovery system 300 may be applied to multi-waste processing (MWP) associated with processing waste, wastewater, and the like included in a container, a chamber, or containment assemblies. For example, the water recovery system 300 may include aspects of a multi-waste processor disclosed in US Non-Provisional Application 17/226,381 filed on April 9, 2021, titled "Multi waste processor," which is hereby incorporated by reference in its entirety. For example, the water recovery system 300 may include and apply heating elements 370 in combination with the features supported by the piezoelectric transducers 315. In the example of FIG. 3, the TCPS 301 includes a receptacle 302. In the example of FIG. 3, the receptacle 302 may be a vacuum chamber. The TCPS 301 may include a door assembly 303, a piston assembly 304, and a dampener assembly 307.

The door assembly 303 may be opened or closed in association with loading bladders 312 or unloading bladders 312 from the receptacle 302. In some embodiments, the door assembly 303 may include one or more heating elements (not illustrated). In some embodiments, the door assembly 303 may be absent (e.g., not include) heating elements.

The piston assembly 304 may support compaction of the bladder 312. In some embodiments, the piston assembly 304 may compress contents (e.g., bladder 312,) of the receptacle 302 to increase or maintain contact between the bladder 312 and piezoelectric transducers 315 located inside the receptacle 302.

The dampener assembly 307 may support shock absorption and dampening. For example, the dampener assembly 307 may support shock absorption and dampening associated with isolating vibrations of the piezoelectric transducers 315 to the TCPS 301 (e.g., preventing or reducing the vibrations from transferring to other equipment).

The receptacle 302 may include a bladder 312 capable of storing substance 314. Aspects of the present disclosure support implementations in which the substance 314 is human waste (e.g., feces, menses, and vomit, which may contain up to 75% water), human generated trash (e.g., which may contain up to 30% water), or wastewater. In some examples, the receptacle 302 may include a removable storage canister (not illustrated).

In some embodiments, the bladder 312 may be a microporous bag. In an example, based on the pore size of pores of the bladder 312, the bladder 312 is capable of retaining the substance 314 and a liquid form of water. The pore size may support extraction of water droplets 316 recovered from the substance 314, example aspects of which are later described herein. In some examples, the bladder 312 may be a single layer bag or may include multiple layers.

In some embodiments, the bladder 312 may be coated or treated for reducing bacteria cell count. For example, one or more of the bladders 312 described herein may be formed of Expanded polytetrafluoroethylene (ePTFE) and be coated with, for example, a silver based layer. In some embodiments, one or more of the bladders 312 may be a multilayer bag where one of the layers is ePTFE and at least one other layer includes silver similar to the trash bags that the ISS TCPS's employs. In another example, one or more of the bladders 312 described herein may be a multilayer bag having a layer formed of ePTFE and a layer formed of Nafion, similar to what the NASA- Paragon Brine Processor uses in the ISS currently. In some aspects, the coating or treatment of the bladder 312 may support further cleaning of effluents, odor control, mitigating or preventing microorganism growth.

In some embodiments, receptacle 302 may include piezoelectric transducers 315 integrated with one or more inner surfaces of the receptacle 302. In some embodiments, door assembly 303 may include piezoelectric transducers 315 integrated with an inner surface of the door assembly 303. In some embodiments, piston assembly 304 may include piezoelectric transducers 315 integrated with one or more outer surfaces of the piston assembly 304.

The water recovery system 300 may include an amplifier 305, a signal generator 306, a computing device 308, control circuitry 320, relay devices 325, and a power source 335 associated with activating the piezoelectric transducers 315 (e.g., inducing vibration of the piezoelectric transducers 315) in association with water recovery and sterilization as described herein. For example, vibration of piezoelectric transducers 315 included in the piezo arrays 317 may support water recovery and sterilization described herein.

According to one or more embodiments of the present disclosure, any of amplifier 305, signal generator 306, computing device 308, control circuitry 320, relay devices 325, and power source 335 may be integrated with the receptacle 302. In some other embodiments, any of amplifier 305, signal generator 306, computing device 308, control circuitry 320, relay devices 325, and power source 335 may be included in a housing 340 (or respective housings) separate from the receptacle 302.

In some embodiments, the amplifier 305 may be included in a housing of the piston assembly 304. Additionally, or alternatively, the amplifier 305 or another amplifier 305 (not illustrated) may be included in a vehicle, a space station, or the like. The amplifier 305 may be detachably couplable directly to the piezoelectric transducers 315 or indirectly to the piezoelectric transducers 315 (e.g., via a connector implemented at the piezoelectric assemblies 310).

The water recovery system 300 may include a signal generator 306 operable to provide a waveform output representing a waveform. In some embodiments, the amplifier 305 may be a power amplifier operable to receive the waveform output, amplify the waveform output, and provide an amplified output to one or more piezoelectric transducers 315 of the water recovery system 300.

The signal generator 306 may include or be electrically coupled to a computing device 308 configured to control the signal generator 306. In some examples, the computing device 308 may provide commands or control signals for controlling the signal generator 306 in response to processing executable instructions stored on a memory of the computing device 308. The computing device 308 may include suitable electronics or processors configured to communicate with and/or control components (e.g., receptacle 302, door assembly 303, piston assembly 304, amplifier 305, signal generator 306, computing device 308, piezoelectric assemblies 310, piezoelectric transducers 315, relay devices 325, power source 335) described herein. According to one or more embodiments of the present disclosure, the computing device 308 may include or be integrated with control circuitry 320 for providing commands or control signals described herein.

In some aspects, the computing device 308 may control signal parameters (e.g., current level, voltage level, frequency, signal type, and the like) of the signal generator 306 based on a target configuration associated with recovering water (e.g., water droplets 316) from a substance 314 (e.g., waste) included in the receptacle 302. For example, the target configuration may be based on characteristics of the substance 314.

Non-limiting examples of the characteristics of the substance 314, based on which the water recovery system 300 may implement the water recovery and sterilization techniques described herein, include a type (e.g., urine, urine brine, feces, trash, menses, emesis, wastewater) of the substance 314 and an estimated water content associated with the substance 314. For example, the water recovery system 300 may configure the signal amplitude and frequency provided by signal generator 306 and amplifier 305, based on the type of the substance 314 and estimated water content (e.g., 75% for human waste, 30% for trash, and the like).

In some aspects, the target water recovery and sterilization parameters (also referred to herein as target criteria for water recovery and sterilization) may include a target temporal duration for water recovery and sterilization. In some aspects, the target dust water recovery and sterilization parameters may be based on a characterization as part of a development phase, in which target levels and behavior for key metrics/variables are determined for conditions equal to or similar to a target substance 314 from which the water recovery system 300 is to recover water.

The power source 335 may power the water recovery system 300 and, in some cases, functionality associated with the TCPS 301. In some embodiments, the power source 335 may power functionality associated with a space station, vehicle, or the like in which the power source 335 is included or to which the power source 335 is electrically coupled.

In some embodiments, the amplifier 305 may reside inside of the housing 340 and interface/connect to piezo arrays 317 residing within or coupled to the receptacle 302. For example, the amplifier 305 may electrically interface with the piezoelectric assemblies 310 (and associated piezoelectric transducers 315 and piezo arrays 317) via one or more connector interfaces located at the receptacle 302. For the example in which the amplifier 305 is exterior to the receptacle 302 (e.g., included in a vehicle, a space station, or the like), the power source 335 may power the water recovery system 300 and, in some cases, other functionality (e.g., functions of the vehicle, the space station, or the like).

The water recovery system 300 may include piezo arrays 317 respective to different parts (e.g., an inner surface) of the receptacle 302 and/or the piston assembly 304. In some embodiments, the piezoelectric assemblies 310 (and respective piezo arrays 317 of piezoelectric transducers 315) may be embedded in fabric or a flexible material removably coupled to an inner surface of the receptacle 302. In some other examples, the piezoelectric assemblies 310 (and respective piezo arrays 317 of piezoelectric transducers 315) may be coupled to or embedded with a surface of the piston assembly 304.

In some examples, the amplifier 305 may electrically interface with the piezo arrays 317 at via a single connector interface common to the piezo arrays 317. Additionally, or alternatively, the amplifier 305 may electrically interface with the piezo arrays 317 via respective connector interfaces of the piezo arrays 317 (or respective connector interfaces of groups of piezo arrays 317). The water recovery system 300 may support vibrating all piezoelectric transducers 315 (e.g., at all piezo arrays 317) of the receptacle 302 in unison. In some other embodiments, the water recovery system 300 may vibrate piezoelectric transducers 315 of different piezo arrays 317 according to a target configuration, a target order, or a target location, example aspects of which are later described herein.

According to one or more embodiments of the present disclosure, based on a magnitude and frequency of signals provided by the amplifier 305, the piezoelectric transducers 315 may vibrate at one or more target frequencies (e.g., one or more ultrasonic frequencies or frequency ranges described herein) supportive of removing water (e.g., water droplets 316) from substance 314 and sterilizing the water. In an example, the water recovery system 300 is configured to extract a cool mist form (e.g., water droplets 316) of the water from the substance, by the vibration of the piezoelectric transducers 315 according to a first frequency.

In another example, the water recovery system 300 is configured to return the water droplets 316 to a liquid state. In some embodiments, the water recovery system 300 is configured to capture the water droplets 316 in a capture tank 345 (e.g., a condensation capture tank, also referred to herein as a storage container) (not illustrated).

In some embodiments, the computing device 308 and amplifier 305 may provide AC voltage signals of different magnitudes and/or frequencies to different sets of piezoelectric transducers 315.

In an example, in response to a first AC voltage signal of a first magnitude and/or first frequency, piezoelectric transducers 315 driven by the first AC voltage signal may vibrate such that that vibration removes water (e.g., water droplets 316) from substance 314. Further, for example, in response to a second AC voltage signal of a second magnitude and/or second frequency, piezoelectric transducers 315 driven by the second AC voltage signal may vibrate such that that vibration sterilizes the recovered water (e.g., water droplets 316, water recovered in a capture tank 345, or the like).

Additionally, or alternatively, the computing device 308 and amplifier 305 may provide an AC voltage signal of a given magnitude and frequency to a set of piezoelectric transducers 315, and in response to the AC voltage signal, the set of piezoelectric transducers 315 may vibrate such that that vibration both removes water (e.g., water droplets 316) from substance 314 and sterilizes the recovered water at the same time.

In some embodiments, the water recovery and sterilization techniques described herein may be implemented as a standalone technique for water recovery and sterilization. Some additional and/or alternative embodiments of the present disclosure support implementation of the described water recovery and sterilization techniques (e.g., vibration using piezoelectric transducers 315) in combination one or more other water processing techniques. For example, the water recovery system 300 may include a processing assembly such as, for example, a urine processing assembly (UPA), water processing assembly (WPA), or other fluid processing assembly suitable for further processing (e.g., sterilization) of the captured water droplets 316 (or liquid form of the water). In an example, the water recovery system 300 may be configured to provide the captured water droplets 316 (or liquid form of the water) to the processing assembly, via tubing 318 (also referred to herein as ducting), ports, and the like.

According to one or more embodiments or applications of the piezoelectric transducers 315 described herein include filters supportive of increasing the lifespan, reducing maintenance time, minimizing consumables, and increasing efficiency of equipment by vibrating out solid residue that may accumulate in the equipment and/or facilitating the extraction of water that might otherwise be entrained in the equipment.

In an example case, the water recovery system 300 may be integrated with a spacesuit water membrane evaporator (SWME), and/or a mini membrane evaporator (supportive of declogging, higher and more effective cooling, desaturation of entrained water, sterilization of remaining solids, and the like), and/or an external layer of the space suit configured for space and/or terrestrial applications. For example, for space applications due to the complexity and cost of maintenance and the cost of spare equipment (e.g., financial cost, cost associated with spatial constraints aboard a space craft, and the like) The systems and techniques described herein support "hands off' approaches for desaturating/unclogging said assemblies (e.g., SWME, mini membrane evaporators, and the like) while providing improved convenience, reduced complexity, and reduced cost. Some SWMEs cool the temperature control loop of the space suit by evaporation of water thru thin micro porous Teflon membrane tubes. The exterior of the membrane is exposed to vacuum thru an exhaust valve. Aspects of the present disclosure support incorporating piezoelectric assemblies 310 and piezoelectric transducers 315 in association with desaturating/unclogging the tubes, sterilizing remaining solid matter, and the like as described herein.

Aspects of the present disclosure support integrating aspects of the water recovery system 300 (e.g., piezoelectric assemblies 310, piezoelectric transducers 315) at filters, tanks, chambers, pump-condensers, and the like of any suitable processing assembly (e.g., MWP, SWME, mini membrane evaporator, and the like) in association with extracting water, sterilizing water, sterilizing remaining solid matter, and/or breaking up remaining solid matter.

In some aspects, for space stations, habitats, and/or vehicles, a MWP may be capable of processing all waste together, which may eliminate multiple separate systems (e.g., TCPS, UPA, WPA, BP, fecal processor). Aspects of the present disclosure support integrating controllable features of the water recovery system 300 (e.g., piezoelectric assemblies 310, piezoelectric transducers 315, vacuum pump 360, heating elements 370, and the like) with one or more MWPs sized for a given application (e.g., space suit, space station, habitat, vehicle), which may provide the features of the multiple separate systems but at a reduced cost, reduced complexity, reduced volume, and improved performance. In some examples, aspects of the present disclosure support integrating features of the water recovery system 300 with particulate filters, beds, tanks, reactors, heat exchangers, and the like.

According to one or more embodiments of the present disclosure, the water recovery system 300 may, using the vibration generated by the piezoelectric transducers 315, lead water droplets 316 to the tubing 318.

According to one or more embodiments of the present disclosure, the water recovery system 300 may support one or more water recovery and sterilization configurations. For example, the relay devices 325 (e.g., solid-state relays, electromechanical relays, or the like) may each be assigned to a piezo array 317 corresponding to a target portion of the TCPS 301. Non-limiting examples of target portions include an inner side surface (or surfaces) of the receptacle 302 and a surface (or surfaces) of the piston assembly 304.

The computing device 308 (e.g., using control circuitry 320) may cycle through different vibrating surfaces according to a target order. In some aspects, the target order may support effective water recovery and sterilization compared to other candidate orders supported by the water recovery system 300. In an example, a target order may include vibrating surfaces associated with an upper portion of the TCPS 301 (e.g., upper inner surface of the receptacle 302), vibrating surfaces associated with a middle portion of the TCPS 301 (e.g., inner side surfaces of the receptacle 302), and vibrating surfaces associated with a lower portion of the TCPS 301 (e.g., lower inner surface of the receptacle 302, piston assembly 304).

The water recovery system 300 may include piezoelectric assemblies 310 (and associated piezoelectric transducers 315) other than the piezoelectric assemblies 310 (and associated piezoelectric transducers 315) illustrated at FIG. 3. For example, the water recovery system 300 may include or implement piezoelectric transducers 315 at surfaces of the tubing 318 or the capture tank 345, and the water recovery system 300 may control the piezoelectric transducers 315 to vibrate in association with sterilizing water (e.g., water droplets 316) as the water passes through the tubing 318 or while the water is stored in the capture tank 345.

In some aspects, the target order may be based on one or more water recovery and sterilization profiles (also referred to herein as water recovery and sterilization configurations) supported by the water recovery system 300. In some examples, the water recovery system 300 may include a water recovery and sterilization profile associated with a relatively highest effectiveness among different water recovery and sterilization profiles. In another example, the water recovery system 300 may include a water recovery and sterilization profile associated with a relatively lowest power consumption (e.g., in exchange for a reduced effectiveness) among different water recovery and sterilization profiles.

Each of the water recovery and sterilization profiles may be associated with activating target piezoelectric transducers 315 of the water recovery system 300. For example, a first water recovery and sterilization profile may be associated with activating target piezoelectric transducers 315 of piezo array 317-a. In another example, a second water recovery and sterilization profile may be associated with activating target piezoelectric transducers 315 of piezo array 317-b. In another example, a third water recovery and sterilization profile may be associated with activating target piezoelectric transducers 315 of all piezo arrays 317 (e.g., for the entire receptacle 302 and for the piston assembly 304).

Accordingly, for example, for a water recovery and sterilization profile in which target piezoelectric transducers 315 (rather than all piezoelectric transducers 315) are activated and remaining piezoelectric transducers 315 are inactive (e.g., a dormant state), the water recovery system 300 may support effective water recovery and sterilization while conserving power. In some embodiments, the control circuitry 320 may be an FPGA based controller supportive of controlling the piezoelectric transducers 315 according to the techniques described herein. In some other embodiments, the water recovery system 300 may support user commands for executing, modifying, and creating water recovery and sterilization profiles described herein. In some aspects, the water recovery and sterilization profiles and associated configurations for activating piezoelectric transducers 315 may be stored in a memory of the computing device 308.

In some embodiments, the water recovery system 300 may include or be associated with other systems 350 and sensor devices 355 for performing other functions separately or in combination with the water recovery system 300, example aspects of which are later described herein. For example, other systems 350 and sensor devices 355 included in or associated with the water recovery system 300 may include a thermoelectric heat pump and heat sink. In some alternative and/or additional examples, the other systems 350 and sensor devices 355 may include a heat exchanger setup including assemblies similar or equivalent to an avionics air assembly (AAA) and/or a medium temperature loop (MTL)/ low temperature loop (LTL) water and heater(s) and vacuum capabilities. In some cases, the water recovery system 300 may provide the vacuum capabilities from a vacuum pump 360 and/or by accessing space vacuum through the proper space station infrastructure 365 (e.g., vacuum exhaust system (VES) in the ISS). In some alternative and/or additional examples, the other systems 350 and sensor devices 355 may include fans, humidity and air temperature sensors, Peltier coolers, T.C., capture tank 345, heaters, vacuum pump. In some alternative and/or additional examples, the other systems 350 may include a processing assembly (e.g., UPA, WPA, or subsystems (for example, catalytic Oxidation Reactor) of the UPA or WPA) in combination with ducting suitable for routing recovered substances (e.g., recovered water, recovered water droplets 316) to the processing assembly.

In an embodiment, the water recovery system 300 may combine heaters, vacuum capabilities (whether a vacuum pump 360 or tapping into the VES for instance) with the piezoelectric assemblies 310 and piezoelectric transducers 315 in order to achieve higher rates of water extraction and sterilization. For example, the heating, vacuuming, and vibration (by piezoelectric transducers 315) may be implemented by the water recovery system 300 to complement each other. For example, through the use of the piezoelectric transducers 315, vacuum pump 360, infrastructure 365, and heating elements 370, the water recovery system 300 may support system redundancy and operational flexibility. For example, for the case of operational failure of heating elements 370, the water recovery system 300 may use piezoelectric transducers 315 for water recovery as described herein (and vice versa). In some embodiments, the water recovery system 300 may implement combined scenarios utilizing vibration (by piezoelectric transducers 315), heating (by heating elements 370), and vacuuming (by vacuum pump 360 or infrastructure 365), but at reduced capacity of at least one of the piezoelectric transducers 315, heating elements 370, and vacuum pump 360 (or infrastructure 365) based on respective availability, target power specifications, or a target time duration for water recovery operations.

In an example embodiment of the water recovery system 300, the piezos operational area = internal surface area of receptacle 302 (Internal TCPS Chamber) = about 8.75 inches x about 8.75 inches x about 11.5 inches deep = 880 inches² =0.57 m². The vibrating action resulting from the piezo setup of the piezoelectric transducers 315 may halve at least the mass heating up time and thus, in combination with heating, may double the water extraction rate of heating alone. It is to be understood that the techniques described herein may be implemented with the piezoelectric transducers 315 described herein, without heating elements or with heating elements (e.g., in a lower power configuration in which heating elements are coupled with piezoelectric transducers 315).

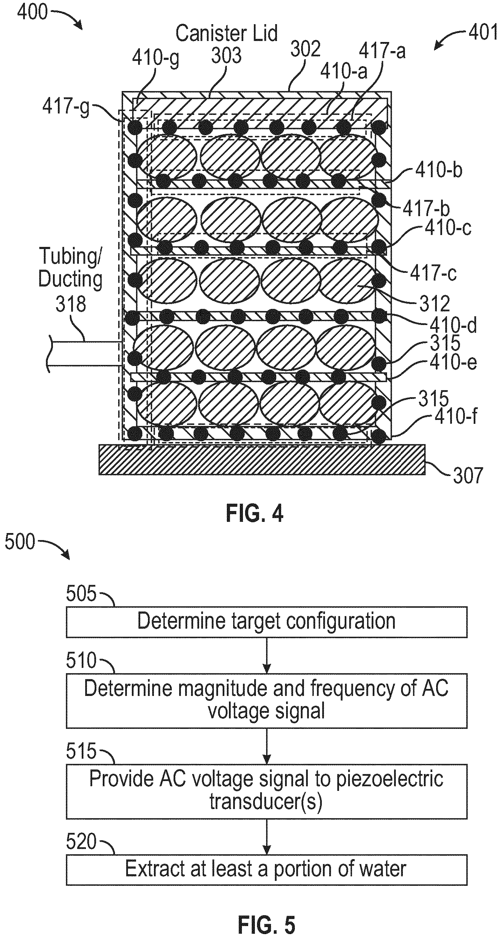

FIG. 4 illustrates an example of a water recovery system 400 supportive of water recovery and sterilization in accordance with one or more embodiments of the present disclosure. The water recovery system 400 includes aspects of piezoelectric system 100 and water recovery system 300 described herein, and repeated descriptions of like elements are omitted for brevity. In some embodiments, the water recovery system 400 may include further features or omit some features of water recovery system 300.

Referring to the example of FIG. 4, the water recovery system 400 includes a TCPS 401. The TCPS 401 includes a receptacle 302, a door assembly 303, bladder 312, and a dampener assembly 307. In some embodiments, (not illustrated), the TCPS 401 may include a piston assembly (e.g., piston assembly 304 illustrated at FIG. 3).

In an embodiment, the TCPS 401 includes multiple piezoelectric assemblies 410, each including a respective piezo array 417 of piezoelectric transducers 415. In the example of FIG. 4, piezoelectric assembly 410-a (including piezo array 417-a) through piezoelectric assembly 410-g (including piezo array 417-g) are labeled and illustrated, but it is to be understood that TCPS 401 may include one or more piezoelectric assemblies 410 additional and/or alternative to the piezoelectric assemblies 410 illustrated therein.

In an example, the TCPS 401 may include one or more piezoelectric assemblies 410 implemented as separator sheets for providing separation between two or more bladders 312 and for providing vibration described herein. For example, referring to FIG. 4, the TCPS 401 includes piezoelectric assembly 410-b and piezoelectric assembly 410-c, in which piezoelectric assembly 410-b and piezoelectric assembly 410-c each include a flexible material. In the example, piezoelectric assembly 410-b and piezoelectric assembly 410-c may provide separation between rows (and/or columns (not illustrated)) of bladders 312.

In some other embodiments, (not illustrated), the TCPS 401 may include one or more piezoelectric assemblies 410 for providing separation between two or more bladders 312 and for providing vibration described herein, and further, may include one or more non-piezoelectric separator sheets (e.g., absent any piezoelectric transducers 315) for further providing separation between two or more bladders 312. It is to be understood that the systems and techniques described herein support any quantity and combination of piezoelectric assemblies 410 as separator sheets and non-piezoelectric separator sheets.

In the example of FIG. 4, a top row of flexible bladders 312 is between piezoelectric assembly 410-b and an upper inner surface (associated with piezoelectric assembly 410-a) of the receptacle 302. Further, for example, the second most top row of flexible bladders 312 is between piezoelectric assembly 410-b and piezoelectric assembly 410-c. In another example, a bottom row of flexible bladders 312 is between piezoelectric assembly 410-e and an lower inner surface (associated with piezoelectric assembly 410-f) of the receptacle 302.

In some embodiments, the computing device 308 (e.g., using control circuitry 320) may cycle through different vibrating surfaces according to a target order. In some aspects, the target order may support effective water recovery and sterilization compared to other candidate orders supported by the water recovery system 400. In an example, a target order may include sequentially vibrating a surface associated with an upper portion of the TCPS 301 (e.g., piezoelectric assembly 410-a (at upper inner surface of the receptacle 302)), a second most upper portion of the TCPS 301 (e.g., piezoelectric assembly 410-b and/or an uppermost portion of piezo array 417-g spanning between piezoelectric assembly 410-a and piezoelectric assembly 410-b), down to a surface associated with a lowest portion of the TCPS 301 (e.g., piezoelectric assembly 410-f and/or a lowermost portion of piezo array 417-g spanning between piezoelectric assembly 410-e and piezoelectric assembly 410-f).

In some other embodiments, the computing device 308 (e.g., using control circuitry 320) may control different vibrating surfaces according to a target configuration such that, for example, piezoelectric transducers 415 of two or more piezoelectric assemblies 410 (or two or more piezo arrays 417) vibrate. For example, the computing device 308 may control the vibration based on an AC voltage signal as described herein and/or through the use of relay devices 325.

In an example, for space station applications, the one or more piezoelectric assemblies 410 may be connected to two main configurations based on the architecture of the vehicle. For example, for a space station, habitat, and/or vehicle, the voltage may be 24 VDC or 120 VDC, with a range of about +/- 4 VDC for both voltage levels. Some embodiments may be dedicated to each input source. Some other embodiments could be adaptable/adjustable where it can accept different input levels and as a result it produces a different output. For example, higher frequencies, may achieve higher water extraction rates, faster dust removal rates, and/or increased sterilization rates.

In some embodiments, the TCPS 401 may include a piston assembly (e.g., piston assembly 304 of FIG. 3) that supports compaction of the bladders 312. In some embodiments, the piston assembly may compress contents (e.g., bladders 312, piezoelectric assemblies 410 (separator sheets), and the like) of the receptacle 302 to increase or maintain contact between the bladders 312 and piezoelectric transducers 415 located on inner surfaces of the receptacle 302 and piezoelectric transducers 415 integrated with separator sheets (e.g., piezoelectric assembly 410-b, piezoelectric assembly 410-c, and the like).

According to one or more embodiments of the present disclosure, piezoelectric transducers described herein (e.g., piezoelectric transducers 315) contract & expand when voltage is applied. The piezoelectric transducers 315 are plugged in to an amplifier (e.g., amplifier 305) making the transducers vibrate at a relatively high frequency, resulting in a colder mist of tiny, atomized water droplets (e.g., water droplets 316) that leave via the micropores of a bag/bladder/membrane (e.g., bladder 312). In some embodiments, piezo patterns may be integrated into the membranes, bladder, or bag, or separately added. In some embodiments, the amplifier may be internal or external to the surface or structure to which a piezo assembly (e.g., piezoelectric assembly 110) is added.

Characterization may be performed as part of a development phase to determine target levels and behavior for key metrics/variables, with as close as possible (e.g., within a threshold tolerance) to the effluent and resulting solids remainders and at the target environmental conditions. Inputs and outputs, characteristics and behavior may differ based on application. For example, the inputs and outputs, characteristics, and behavior may respectively differ for a wastewater membrane, a filter, a metallic heat exchanger, and a condenser.

The systems and techniques described herein enable reduced post processing and cleaning compared to other techniques. For example, the water recovery and sterilization techniques described herein may be implemented with less processing time compared to other techniques which may use temperature differentials across a membrane/bag/bladders to draw water content from contained waste contained in the membrane/bag/bladders. The systems and techniques described herein provide an atomization effect induced by vibration of the described piezoelectric transducers. In some aspects, the water recovery and sterilization techniques described herein may be used in combination with one or more other processing techniques (e.g., techniques using temperature differentials across a membrane/bag/bladder to draw water content).

The water recovery and sterilization techniques described herein provide advantages over some other water recovery techniques. For example, by vibrating a substance 314 (e.g., human waste, trash, plastic, and the like) in association with extracting a resultant cool mist (e.g., water droplets 316), the techniques described herein avoid instances in which the substance 314 reaches a temperature at which gas contaminants would otherwise be released. Accordingly, for example, the water recovery and sterilization techniques described herein support increased safety compared to other techniques.

In some aspects, the water recovery and sterilization techniques described herein support reduced size of waste storage canisters (e.g., receptacle 302), which may support reduced overhead costs (e.g., space, fuel, manufacturing, and the like) for storing waste aboard a spacecraft, a space station, or the like.

Example implementation aspects of the water recovery system 300, the TCPS 301, and the receptacle 302 as supported by the present disclosure are described herein.

Piezoelectric interface Operating Area = receptacle 302 with internal cylindrical shape of ~ 6" dia. x 12" deep, including 4 circular separator sheets (e.g., piezoelectric assemblies 410) of 6" diameter each = 283 in² + 4* 28 in² = 395 in² = (up to) 0.25 m². Based on Sim feces testing, a board of 50 piezo transducers "120 x 80 mm" [ 0.0096 m² ~15 in² ] at 27.5 V can remove approx. 9.74 g/hr; and that average drying rate is directly proportional to piezo m², therefore: 0.25/0.0096~(up to) 26 times faster or an approx. rate - 26* 9.74g/hr. -(up to) 253 g/hr, thus removing 90% of the water content of 18 bags each at about 125 g, 75% water, yields ~1519 g of water to be removed, which means that the example configuration could remove the described amount of water in about 6 hrs for cases in which all piezoelectric transducers 415 are engaged in unison, which may or may not be feasible due to power constraints. In some embodiments, the techniques described herein may include waste processing as waste is collected, thus supporting a receptacle 302 for fecal processing, in which the receptacle 302 has a relatively smaller size compared to the current waste canister in the toilet of the ISS. The current waste canister in the ISS is 6" dia. x 7" deep (188 in²) with only 2 separator sheets ~56 in2. ~244 in2 ~0.16 m² , and the current waste canister in the ISS is absent any piezoelectric transducer solution as described herein. According to one or more embodiments of the present disclosure, the techniques described herein support a possible water extraction rate of up to 0.16/0.25 * 253g/ hr ~ 162 g/hr, which results in about 9.4 hrs drying time for about 18 feces filled bags.

Using an example average of 2 defecation per day per person x 4 crew member, then a small canister (e.g., 6" diameter x 7" deep) would get full in around 2 days, thus processing time less than that is appropriate; therefore even the smallest canister is capable of handling other waste such as for instance vomit, which is nominally collected also in fecal microporous bags, and it could happen up to a max of 1.5L per instance based on current NASA' requirements. However, in some cases, other waste (e.g., vomit) may generally average smaller volumes such as, for example, 0.5L.

The water recovery system 300 may support a bigger canister scenario such as, for example, for a combined toilet waste processor. The combined toilet-waste processor may support the collection of urine, feces, vomit, and menses in microporous membrane bags as described herein, and the placed inside said canister with piezo interfaces with or without heaters. In some cases, 1 urination event can be as much as 1L. However, on average in the ISS: ~ 0.35 mg/ instance x 4 crew member x 6 urinations/day x 0.9 recovery x 0.95% water content- 7200 g/day. Using the calculation described herein, a configuration of the receptacle 302 of 6" diameter x 12" deep may support a water extraction rate of up to 253 g/hr. In an example, with such a water extraction rate, the water recovery system 300 may take ≤ 29 hrs for the urine to be dried with this configuration, plus ≤ 3 hrs to process the daily astronauts feces. For this combined waste case, the receptacle 302 (toilet canister) may be at least : (32/24 ~ 1.33) bigger to take care of all daily toilet waste; like for instance: 6" diameter & 16" deep if using the piezoelectric by themselves to extract the water from the waste. In some embodiments, if a combined configuration of heating plus vacuum pump is implemented, as to maintain water boiloff conditions (e.g., 2 psia and 65 degrees C), water boiloff rates could significantly increase the overall water recovery rates (e.g., > 50% increase ) vs the water extraction rates using piezo configuration alone. In such case, a smaller canister again could be employed like for instance ( 6" diameter x 12" deep).

Even for the 4 consecutive defecation scenario in [18] resulting in 125g*75% water* 90% extraction*4 bags ~338g of water to remove; bags in the bottom of the canister will only be under the close influence of the equivalent of about a 6" dia. cylinder with 2" depth worth of piezo area, which translates to about: 94 in2 ~0.06 m2 yielding- 0.06/.0096*9.74 g/hr ~61 g/hr; so, water extraction would take-5.5 hrs in this worse case defecation event.

Aspects of the piezoelectric based implementations described herein may support treatment of human generated trash. For example, trash in space (e.g., up to 30% water) is being stored in bags, later stacked in a vehicle/station and eventually disposed of upon reentry into the Earth's atmosphere. It is an immediate NASA goal to recover 90% of water from accumulated trash aboard a space station, a space exploration vehicle, habitats, and the like, and reuse the solids as raw material.

By using an amplifier (e.g., amplifier 305), driven transducers (e.g., piezoelectric transducers 315) are made to vibrate at a relatively high frequency navigating the water (e.g., as water droplets 316) closer to the pores of a storage bag (e.g., bladder 312) including trash solid matter and gradually removing the water from the trash solid matter while also sterilizing the water. The systems and techniques described herein support interweaving the piezoelectric transducers 315 (or piezoelectric assemblies 310 described herein) in the bag itself or attaching the piezoelectric transducers 315 (or piezoelectric assemblies 310) the chamber' internal wall in contact with the trash filled bag.

The techniques described herein advantageously combine a piezo interface inside of a trash processor and a microporous bags/bladder. The piezo-based water recovery and sterilization techniques described herein support the processing and sterilization of trash and water extracted from the trash. In some aspects, the piezo-based water recovery and sterilization techniques described herein may be incorporated with heat/partial vacuum/compaction techniques for trash processing for further expediting trash processing or increasing a sterilization efficacy associated with trash processing.

Aspects of the piezoelectric based implementations described herein may support treatment and processing of wastewater, and in some aspects, water recovery from wastewater and removal of wastewater residue.

Wastewater residue accumulating in membranes, heat exchangers, condensers, meshes, ducting and pipes, chambers, sieves, traps, filters, and the like, and overall wastewater management systems and devices is a documented issue on Earth and space applications.

Some techniques for mitigating issues of wastewater residue include flushing with liquid (e.g., clean water or some suitable application based chemical solution) or gas (e.g., clean air or an inert gas), vacuuming, disassembly plus chemical cleaning, brushing off residue with tools, utilizing small maintenance robots, disinfection (e.g., with UV light or heat). Such techniques may be relatively time consuming or CONOPS sensitive, expensive, and/or impractical for space exploration.

Solid wastewater residue is challenging to safely and effectively remove in the space environment due to the small size of the solid particles and the microgravity environment. In some cases, since some payload or devices for waste processing are fairly compact, the solid particles (e.g., due to particle size) may end up in crevices of equipment which are unable to be fully cleaned or flushed of the particles. In some cases, the solids are organic based or derived from organic matter such as, for example, food, feces, urine, oil/lubricants, vomit, menses, and the like. In some other cases the solids are inorganic from sources such, for example, plastic, rubber, epoxy, metallic, fabrics, or the like.

Some wastewater processing techniques inherently involve recovering the processed effluent, which may add humidity to the equation. Accumulation of solid organic particles in a humid environment portions of a system which are physically inaccessible or difficult to access is a difficult yet common problem, and the accumulation of such particles may have several impacts on key characteristics (e.g., delta pressure, microbial growth, flowrate, and efficiency) of a waste processing system. The water recovery and sterilization techniques described herein may be implemented alone or in combination with already established approaches for wastewater processing.

According to one or more embodiments of the present disclosure, by using an amplifier (e.g., amplifier 105, amplifier 305), transducers (e.g., piezoelectric transducers 115, piezoelectric transducers 315 described herein) may be driven and controlled to vibrate at a relatively high frequency, in which the vibration is capable of removing foreign objects from a surface to be cleaned.

The piezoelectric transducers (e.g., piezoelectric transducers 115, piezoelectric transducers 315) and/or associated assemblies (e.g., piezoelectric assembly 110, piezoelectric assemblies 310) may be interwoven in the surface or membrane itself to be cleaned. In some aspects, the amplifier may be built-in within the structure to be cleaned.

For example, the amplifier may be built-in or integrated with a reverse osmosis membrane to minimize saturation and extend useful life while minimizing maintenance, filters and condensers. In an example, based on a trigger condition (e.g., user input, user preference, or the like), the waste dust can be vibrated out by the controlled vibration of the piezoelectric transducers. Additionally, or alternatively, a similar piezoelectric based assembly could be added external or internal of the surface or desired membrane that the user desires to rid of solid greywater remainders.

According to one or more embodiments of the present disclosure, the systems and techniques described herein may control the vibration of the piezoelectric transducers such that the vibration is capable of effectively reducing the microbial count of a contaminated area (e.g., a surface to be cleaned of solid greywater remainders) for example, by more than 5000 times.

In some embodiments, the systems and techniques described herein may be applied to a condenser or osmosis membrane. For example, the systems and techniques described herein may integrate a piezo-based interface (e.g., a piezoelectric assembly 110 described herein) integrated into a heat exchanger, and controlled vibration of the piezoelectric transducers may remove or break down solid greywater remainders. In some aspects, the controlled vibration may sterilize the solid greywater remainders.

The systems and techniques described herein may be implemented as a standalone package and/or may be integrated into membranes, heat exchangers, condensers, meshes, ducting and pipes, chambers, sieves, traps, filters, or the like, providing advantages of operational flexibility. For example, the systems and techniques for wastewater removal, wastewater treatment, wastewater residue removal and treatment may be implemented in a wastewater management system or device. For example, the systems and techniques described herein may be implemented standalone or in combination with other techniques which utilize vacuum, flushing, wiping, brushing, filtration, using nanocoated layers or surfaces or a combination thereof, to mitigate the residue buildup problem. Utilizing a piezoelectric interface to force vibration that breaks up and facilitate the removal of solid residue buildup, while at the same time disinfecting the solids, provides improved removal and sterilization compared to other techniques.

The systems and techniques for wastewater residue mitigation and removal as described herein may support reduced over costs associated with space stations, planetary habitats, space exploration vehicles, and the like. For example, the systems and techniques described herein (e.g., through the removal and sterilization of solid residue buildup) may extend the life expectancy of equipment, which may thereby reduce equipment costs for spare equipment and consumables and reduce overall maintenance costs.

Characterization may be performed as part of a development phase to determine target levels and behavior for key metrics/variables, with as close as possible (e.g., within a threshold tolerance) to the effluent and resulting solids remainders and at the target environmental conditions. Inputs and outputs, characteristics and behavior may differ based on application. For example, the inputs and outputs, characteristics, and behavior may respectively differ for a wastewater membrane, a filter, a metallic heat exchanger, and a condenser.

In an example referring to FIG. 2, the piezoelectric assembly 110 may be formed different shapes and forms. For example, the piezoelectric assembly 110 may be configured in a sleeve configuration supportive of cylindrical shaped end-product applications. In an example, the piezoelectric assembly 110 may be configured as a cylindrical shaped section (e.g., tubing) of a heat exchanger or condenser. In some example implementations, the piezoelectric assembly 110 may be installed interior or exterior to tubing (e.g., inlet tubing, outlet tubing, tubing of heat exchanger coils, tubes associated with a fluid path, or the like) of the heat exchanger. In an example, the piezoelectric assembly 110 may include piezoelectric transducers 115 capable of vibrating at a frequency associated with removing solid wastewater residue from the tubing of the heat exchanger. In some aspects, the vibration of the piezoelectric transducers 115 may reduce foaming or bubbling of effluent of a feed effluent heat changer (FEHE).

FIG. 5 illustrates an example flowchart of a method 500 in accordance with one or more embodiments of the present disclosure. The method 500 may be implemented by the example aspects of a water recovery system 300 described herein.

At 505, the method 500 includes determining a target configuration associated with recovering water from a substance comprised in a receptacle. At 510, the method 500 includes determining, based on the target configuration, a magnitude and a frequency of an alternating-current (AC) voltage signal to be applied to one or more piezoelectric transducers of a plurality of piezoelectric transducers, wherein the plurality of piezoelectric transducers are comprised in the receptacle. At 515, the method 500 includes providing, by amplifier circuitry, the AC voltage signal to the one or more piezoelectric transducers, based on the target configuration, wherein the one or more piezoelectric transducers vibrate based on the magnitude and the frequency of the AC voltage signal, wherein the vibration of the one or more piezoelectric transducers separates the water from the substance. At 520, the method 500 includes extracting, to at least one storage container, at least a portion of the water separated from the substance.

In the descriptions of the flowcharts herein, the operations may be performed in a different order than the order shown, or the operations may be performed in different orders or at different times. Certain operations may also be left out of the flowcharts, one or more operations may be repeated, or other operations may be added to the flowcharts.

FIGS. 6A through 6C illustrate examples of a water recovery system 600 supportive of water recovery and sterilization in accordance with one or more embodiments of the present disclosure. The water recovery system 600 includes aspects of piezoelectric system 100, water recovery system 300, and water recovery system 400 described herein, and repeated descriptions of like elements (e.g., an amplifier 105) are omitted for brevity. In some aspects, the water recovery system 600 includes a laundry system 601, examples of which are illustrated at FIGS. 6A through 6C.

The laundry system 601 may include aspects of a fluid recovery system and a laundry system disclosed in U.S. Non-provisional Patent Application 17/963,952 filed on Oct 11, 2022, titled "Fluid Recovery Systems For Laundry Systems," which is hereby incorporated by reference in its entirety.

In some embodiments, the laundry system 601 may include a laundry chamber 602, and the laundry chamber 602 may include one or more piezoelectric assemblies 610, each including piezoelectric transducers 615. In some other embodiments, the laundry chamber 602 may include multiple piezoelectric assemblies 610, and each piezoelectric assembly 610 may include piezoelectric transducers 615.

In some embodiments, after the laundry system 601 completes a washing operation (e.g., agitation, or the like), the laundry system 601 may drain "loose" water (e.g., water not entrained within clothing or other content included in the laundry chamber 602) from the laundry chamber 602 using one or more valves or ports integrated with or coupled to the laundry chamber 602. The laundry system 601 may vibrate piezoelectric transducers 615 of the piezoelectric assembly 610 in accordance with the techniques described herein, and the vibration may extract the water entrained within laundry contents (e.g., t-shirts, underwear, towels, and the like) inside of the laundry chamber 602. Accordingly, for example, the water extraction by the piezoelectric transducers 615 of the laundry system 601, in combination with other drying operations (e.g., using heating elements, vacuum elements, or the like) may support increased drying efficiency and reduced drying time.

In some other cases, the water extraction by the piezoelectric transducers 615 of the laundry system 601 may support drying of laundry contents without other drying operations (e.g., using heating elements, vacuum elements, hanging of the laundry contents on a rack located near the environmental control and life support system (ECLSS) vent of a space station, or the like). Accordingly, for example, the laundry system 601 may support increased reduced power consumption (e.g., for cases in which the power associated with vibrating the piezoelectric transducers 615 is less than the power associated with powering the heating elements and/or vacuum elements).

Referring to FIG. 6A, the piezoelectric assembly 610-a may be disposed on an inner surface of the laundry chamber 602, such that the piezoelectric assembly 610-a (and piezoelectric transducers 615) come into contact with clothing 620 during a wash cycle and/or dry cycle of the laundry system 601. For example, the piezoelectric assembly 610-a may be disposed on an inner bottom surface of the laundry chamber 602.

Referring to FIG. 6B, piezoelectric assemblies 610-a and 610-b may respectively be disposed on the inner bottom surface and an inner side surface of the laundry chamber 602, such that the piezoelectric assembly 610-a (and piezoelectric transducers 615) has a relatively large amount of contact with clothing 620 during a wash cycle and/or dry cycle of the laundry system 606. In some aspects, the piezoelectric assembly 610-b has relatively less contact with clothing 620 compared to piezoelectric assembly 610-a but is immediately close to the clothing 620, such that the piezoelectric assembly 610-b is in contact with clothing 620 during portions of the wash cycle and/or dry cycle.

Referring to FIG. 6C, piezoelectric assemblies 610-a, 610-b, 610-c, and 610-d may respectively be disposed on the inner bottom surface, an inner side surface, an inner top surface, and an inner side surface of the laundry chamber 602, and piezoelectric assembly 610-e may be positioned internal to the laundry chamber 602. For example, piezoelectric assembly 610-e may be disposed on or integrated with an agitator 625 of the laundry system 601. Accordingly, for example, one or more of the piezoelectric assemblies 610 (e.g., in some cases, multiple piezoelectric assemblies 610) may be in contact with clothing 620 during portions of the wash cycle and/or dry cycle.

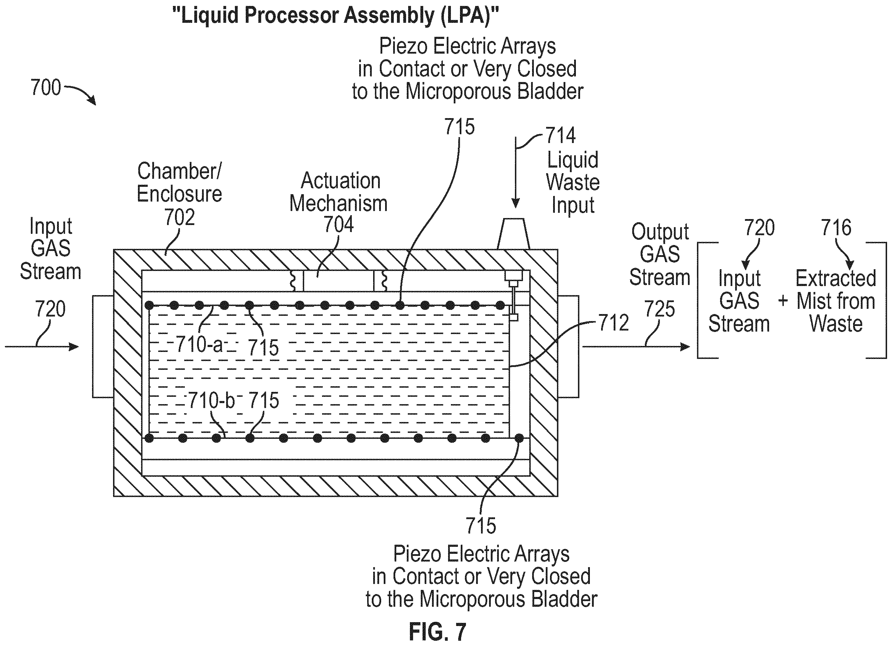

FIG. 7 illustrates an example of a liquid processor assembly 700 supportive of water recovery and sterilization in accordance with one or more embodiments of the present disclosure. The liquid processor assembly 700 includes aspects of piezoelectric system 100, water recovery system 300, and water recovery system 400 described herein, and repeated descriptions of like elements (e.g., an amplifier 105) are omitted for brevity.

In some embodiments, the liquid processor assembly 700 may include a chamber 702, and the chamber 702 may include a piezoelectric assembly 710-a including arrays of piezoelectric transducers 715. In some other embodiments, the chamber 702 may include piezoelectric assemblies 710-a and 710-b respectively disposed on an upper surface and a lower surface of the chamber 702. The liquid processor assembly 700 may include an actuation mechanism 704 and a bladder 712 capable of storing waste 714. Non-limiting examples of the waste 714 include human waste (e.g., feces, menses, and vomit, which may contain up to 75% water), human generated trash (e.g., which may contain up to 30% water), or wastewater.

The actuation mechanism 704 may include aspects of the piston assembly 304 described with reference to FIG. 3.

The liquid processor assembly 700 may include other components described with reference to FIG. 3 in association with controlling the liquid processor assembly 700 and the piezoelectric transducers 315. For brevity, the components (e.g., amplifier 305, signal generator 306, computing device 308, control circuitry 320, relay devices 325, power source 335, other systems 350, sensor devices 355, vacuum pump 360, infrastructure 365, heating elements 370, and the like) are not illustrated, and repeated descriptions of the components are omitted for brevity.

In accordance with example aspects of the present disclosure, the liquid processor assembly 700 may receive an input gas stream 720 and waste 714. The liquid processor assembly 700 may extract, from waste 714, mist 716 (e.g., water droplets) based on vibration by the piezoelectric transducers 715 in accordance with example aspects of the present disclosure. In an example, the output gas stream 725 may include the input gas stream 720 and the extracted mist 716.

In accordance with example aspects of the present disclosure, the actuation mechanism 704 may be a bellows or jack screw mechanical type of mechanism. The actuation mechanism 704 may move the piezoelectric assembly 710-a (and/or the piezoelectric assembly 710-b) in association with approximately matching the water removal rate by the liquid processor assembly 700. For example, as the content (e.g., amount of waste 714) included in the bladder 712 diminishes, the actuation mechanism 704 may move the piezoelectric assembly 710-a (and/or the piezoelectric assemblies 710-b) such that the piezoelectric assemblies 710 (and associated piezoelectric transducers 715) remain in contact with the bladder 712 or remain within a threshold distance (e.g., 0.1 inches) of the bladder 712.

In some embodiments, the liquid processor assembly 700 may be implemented with the piezoelectric assemblies 710 alone or in combination with other equipment (e.g., vacuum, fans/blowers, heaters, or the like) described herein supportive of wastewater extraction, wastewater sterilization, solids extraction, solids sterilization, and the like as described herein, which may provide increased effectiveness in association with the removal and sterilization of water and/or solids compared to other techniques.

In an example, the liquid processor assembly 700 may control the vibration of the piezoelectric transducers 315, and the vibration causes the water content from the waste 714 inside the bladder 712 to exit (in the form of mist 716) via pores of the bladder 712. The mist 716 (in the form of tiny water droplets) will be carried out of the chamber 702 by the input gas stream 720 (sweep) into a post processing component. Non-limiting examples of post processing include filtering, condensating/heat exchange, chemical treatment, UV, electrochemical (e.g., catalytic oxidation reactor bed, electrolysis, coagulation, or the like).

In some examples, the input gas stream 720 may be a heated gas stream or an unheated gas stream. In some embodiments, the liquid processor assembly 700 may implement the piezoelectric transducers 715 in combination with the input gas stream 720 (e.g., a heated gas stream) and/or exposing the bladder 712 to a partial vacuum so as to draw the water content out of the waste 714 via the micropores, which may provide faster water recovery compared to other techniques (e.g., techniques absent use of the piezoelectric transducers 715).

The term "about" is intended to include the degree of error associated with measurement of the particular quantity based upon the equipment available at the time of filing the application.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present disclosure. In addition, many modifications may be made to adapt a particular situation or material to the teachings of the present disclosure without departing from the essential scope thereof. Therefore, it is intended that the present disclosure not be limited to the particular embodiment disclosed as the best mode contemplated for carrying out this present disclosure, but that the present disclosure will include all embodiments falling within the scope of the claims.

The corresponding structures, materials, acts and equivalents of all means or step plus function elements in the claims below are intended to include any structure, material, or act for performing the function in combination with other claimed elements as specifically claimed. The description of the present disclosure has been presented for purposes of illustration and description, but is not intended to be exhaustive or limited to the technical concepts in the form disclosed. Many modifications and variations will be apparent to those of ordinary skill in the art without departing from the scope and spirit of the disclosure. The embodiments were chosen and described in order to best explain the principles of the disclosure and the practical application and to enable others of ordinary skill in the art to understand the disclosure for various embodiments with various modifications as are suited to the particular use contemplated.

While the various embodiments to the disclosure have been described, it will be understood that those skilled in the art, both now and in the future, may make various improvements and enhancements which fall within the scope of the claims which follow. These claims should be construed to maintain the proper protection for the disclosure first described.

## Claims

1. A system comprising:
a receptacle (302);
one or more piezoelectric transducers (315) comprised in the receptacle (302); and
amplifier circuitry coupled to the one or more piezoelectric transducers (315) and configured to provide an alternating-current, AC, voltage signal to the one or more piezoelectric transducers (315), based on a target configuration associated with recovering water from a substance comprised in the receptacle (302),
wherein:
the one or more piezoelectric transducers (315) are configured to vibrate based on a magnitude and a frequency of the AC voltage signal; and
the system is configured to extract a mist form of the water from the substance, based on the vibration of the one or more piezoelectric transducers (315).

2. The system of claim 1, further comprising:
one or more flexible bladders (312) configured to store the substance and comprising a plurality of pores, wherein the one or more flexible bladders (312) are configured to retain a liquid form of water, based on a pore size of the plurality of pores,
wherein the system is configured to extract the mist form of the water via the plurality of pores, based on the vibration of the one or more piezoelectric transducers (315).

3. The system of claim 2, wherein the one or more piezoelectric transducers (315) are removably coupled to one or more inner surfaces of the receptacle (302) and are in contact with one or more surfaces of the one or more flexible bladders (312); or wherein the one or more piezoelectric transducers (315) are integrated with a material of the one or more flexible bladders (312).

4. The system of claim 2 or 3, wherein:
the vibration of the one or more piezoelectric transducers (315) separates the water from the substance; and
at least a portion of the water separated from the substance based on the vibration of the one or more piezoelectric transducers (315) is in a mist form comprising water droplets.

5. The system of any of claims 2-4, further comprising one or more piezoelectric assemblies comprised in the receptacle (302), wherein:
the one or more piezoelectric assemblies comprise the one or more piezoelectric transducers (315);
the one or more piezoelectric assemblies comprise a flexible material; and
the one or more flexible bladders (312) are between the one or more piezoelectric assemblies and at least one inner surface of the receptacle (302).

6. The system of any of claims 2-4, further comprising one or more piezoelectric assemblies comprised in the receptacle (302), wherein:
the one or more piezoelectric assemblies comprise the one or more piezoelectric transducers (315);
the one or more piezoelectric assemblies comprise a flexible material; and
at least one first flexible bladder of the one or more flexible bladders (312) is between the one or more piezoelectric assemblies and at least one inner surface of the receptacle (302), and optionally wherein:
at least one second flexible bladder of the one or more flexible bladders (312) is between the one or more piezoelectric assemblies and at least one second inner surface of the receptacle (302); or
the at least one second flexible bladder is between the one or more piezoelectric assemblies and one or more second piezoelectric assemblies comprised in the receptacle (302), wherein the one or more second piezoelectric assemblies comprise one or more second piezoelectric transducers.

7. The system of any preceding claim , wherein the substance comprises at least one of urine; urine brine; feces; trash; and wastewater.

8. The system of any preceding claim, further comprising:
at least one storage container;
one or more ports coupled between the receptacle (302) and the at least one storage container; and
one or more second piezoelectric transducers coupled to the one or more ports,
wherein:
the system is configured to transfer the recovered water to at least one storage container via the one or more ports;
the amplifier circuitry is configured to provide a second AC voltage signal to the one or more second piezoelectric transducers, based on the target configuration, wherein the one or more second piezoelectric transducers are configured to vibrate based on a second magnitude and a second frequency of the AC voltage signal; and
the vibration of the one or more second piezoelectric transducers sterilizes at least a portion of the recovered water.

9. The system of any preceding claim, wherein the target configuration is based on one or more characteristics of the substance, the one or more characteristics comprising at least one of:
a type of the substance; and
an estimated water content associated with the substance.

10. The system of any preceding claim, wherein:
the target configuration is based on a target sterilization level associated with sterilizing at least a portion of the recovered water; and
the vibration of the one or more piezoelectric transducers (315) sterilizes at least the portion of the water.

11. The system of any preceding claim, wherein:
the target configuration is based on a target sterilization level associated with sterilizing at least a portion of solid matter comprised in the substance; and
the vibration of the one or more piezoelectric transducers (315) sterilizes at least the portion of the solid matter.

12. The system of any preceding claim, wherein the system is configured for recovering the water under partial gravity, microgravity, or zero gravity conditions.

13. A receptacle (302) comprising:
one or more piezoelectric transducers (315); and
amplifier circuitry coupled to the one or more piezoelectric transducers (315) and configured to provide an alternating-current, AC, voltage signal to the one or more piezoelectric transducers (315), based on a target configuration associated with recovering water from a substance comprised in the receptacle (302),
wherein:
the one or more piezoelectric transducers (315) are configured to vibrate based on a magnitude and a frequency of the AC voltage signal; and
the receptacle (302) is configured to extract a mist form of the water from the substance, based on the vibration of the one or more piezoelectric transducers (315).

14. The receptacle (302) of claim 13, further comprising:
one or more flexible bladders (312) configured to store the substance and comprising a plurality of pores, wherein the one or more flexible bladders (312) are configured to retain a liquid form of water, based on a pore size of the plurality of pores,
wherein the receptacle (302) is configured to extract the mist form of the water via the plurality of pores, based on the vibration of the one or more piezoelectric transducers (315), and optionally wherein the one or more piezoelectric transducers (315) are removably coupled to one or more inner surfaces of the receptacle (302) and are in contact with one or more surfaces of the one or more flexible bladders (312); or wherein the one or more piezoelectric transducers (315) are integrated with a material of the one or more flexible bladders (312); and/or wherein:
the vibration of the one or more piezoelectric transducers (315) separates the water from the substance; and
at least a portion of the water separated from the substance based on the vibration of the one or more piezoelectric transducers (315) is in a mist form comprising water droplets.

15. A method comprising:
determining a target configuration associated with recovering water from a substance comprised in a receptacle (302);
determining, based on the target configuration, a magnitude and a frequency of an alternating-current (AC) voltage signal to be applied to one or more piezoelectric transducers (315) of a plurality of piezoelectric transducers (315), wherein the plurality of piezoelectric transducers (315) are comprised in the receptacle (302);
providing, by amplifier circuitry, the AC voltage signal to the one or more piezoelectric transducers (315), based on the target configuration, wherein the one or more piezoelectric transducers (315) vibrate based on the magnitude and the frequency of the AC voltage signal, wherein the vibration of the one or more piezoelectric transducers (315) separates the water from the substance; and
extracting, to at least one storage container, at least a portion of the water separated from the substance.
